# EUROPÄISCHE PATENTSCHRIFT

(11) **EP 0 692 257 B1**
(45) Veröffentlichungstag und Bekanntmachung des Hinweises auf die Patenterteilung: **22.08.2001**
(21) Anmeldenummer: 95111050.1
(22) Anmeldetag: 14.07.1995
(51) Int. Cl.: A61K 35/78

(54) **Verfahren zur Herstellung von pestizidarmen Wirkstoffkonzentraten aus Pflanzen**
Process for the preparation of pesticide-poor concentrates of active components of plants
Procédé de fabrication de concentrés de produits actifs pauvres en pesticides, en provenance de plantes

(30) Priorität: 14.07.1994 CH 225894
(43) Veröffentlichungstag der Anmeldung: 17.01.1996
(73) Patentinhaber: Emil Flachsmann AG, CH-8820 Wädenswil (CH)
(72) Erfinder: Steiner, Rudolf, CH-8806 Bäch (CH); Colombi, Renato, CH-8820 Wädenswil (CH)
(74) Vertreter: Zink-Wild, Markus Peter

(56) Entgegenhaltungen:
- EP-A- 0 261 421
- EP-A- 0 382 116
- DE-B- 1 125 117
- JP-A- 4 182 434

## Beschreibung

Die vorliegende Erfindung betrifft ein Verfahren zur Herstellung von pestizidarmen Wirkstoffkonzentraten aus Pflanzen.

Im Stand der Technik ist kein Verfahren bekannt, mit welchem bestimmte, ausgewählte Pflanzeninhaltsstoffe selektiv und in möglichst reiner und pestizidarmer Form sowie in hoher Konzentration erhältlich wären.

In der DE PS 17 67 098 ist ein Verfahren beschrieben, bei welchem verschiedene Extraktionen ausgeführt werden. Bei diesem Verfahren werden zudem halogenierte Lösungsmittel, wie etwa Methylenchlorid, Chloroform und Tetrachlorkohlenstoff, verwendet.

In der DE OS 39 40 092 ist ein Verfahren beschrieben, bei dem ein Extrakt aus den Blättern von Ginkgo biloba erhalten wird. Dieser Extrakt hat einen erhöhten Gehalt an Flavonglykosiden. Dieses mehrstufige Verfahren ist sehr kompliziert.

In JP-A-04 182 434 wird ein Verfahren zur Herstellung eines Extraktes aus Ginkgo biloba Blättern beschrieben.

Es ist ein Ziel der vorliegenden Erfindung, ein Verfahren zur Verfügung zu stellen, mit welchem bestimmte, ausgewählte Pflanzeninhaltsstoffe selektiv und in möglichst reiner und pestizidarmer Form sowie in hoher Konzentration erhältlich sind.

Die mit dem neuen Verfahren hergestellten Wirkstoffkonzentrate sollen insbesondere allergenarm sein und vorzugsweise keine Alkylphenole enthalten.

Dieses neue Verfahren soll zudem technisch einfach in seiner Ausführung sein.

Bei der Realisierung dieses Verfahrens soll der gesamte Energieverbrauch gering sein.

Dieses Verfahren soll zudem wenig Schritte aufweisen. Ausserdem sollen nur relativ geringe Mengen an organischen Lösungsmitteln benötigt werden.

Völlig überraschend wurde nun ein Verfahren gefunden, mit welchem alle obigen Ziele erreicht und die obigen Nachteile überwunden werden können.

Das erfindungsgemässe Verfahren zur Herstellung von pestizidarmen Wirkstoffkonzentraten aus Pflanzen ist dadurch gekennzeichnet, dass man
- in einem ersten Schritt Pflanzen oder Teile davon, welche im frischen oder getrockneten Zustand eingesetzt werden können, mit wenigstens einem polaren Lösungsmittel bei einem gemessenen pH-Wert im Bereich von 3,2 bis 9,8 derart extrahiert, dass im erhaltenen Primärextrakt hauptsächlich die gewünschten Pflanzeninhaltsstoffe, begleitet von nicht gewünschten Pflanzeninhaltsstoffen, Begleitstoffen und Pestiziden, enthalten sind, wobei im Falle von Ginkgo folia das genannte polare Lösungsmittel wässrig ist und von 0 bis 20 Gew.-% an Alkohol(en) und/oder Keton(en enthält,
- in einem zweiten Schritt den genannten Primärextrakt derart mit Adsorberharz(en) in Kontakt bringt, dass einerseits die gewünschten Pflanzeninhaltsstoffe daran adsorbiert werden, und andererseits die Lösung mit den nicht gewünschten Pflanzeninhaltsstoffen, den Begleitstoffen und Pestiziden vom beladenen Harz separiert wird,
- in einem dritten Schritt die gewünschten Pflanzeninhaltsstoffe vom genannten Harz mit wenigstens einem Lösungsmittel desorbiert werden, und
- in einem vierten Schritt das (die) Lösungsmittel entweder teilweise oder vollständig entfernt und so ein Wirkstoffkonzentrat erhält.

Bevorzugte Ausführungsformen dieses Verfahrens sind in den abhängigen Ansprüchen definiert.

Der Primärextrakt kann gemäss den im Stand der Technik bekannten Verfahren hergestellt werden, beispielsweise mittels Perkolation, Mazeration oder einer dynamischen Extraktion; siehe Marcel Loncin "Die Grundlagen der Verfahrenstechnik in der Lebensmittelindustrie" (1969), insbesondere Seiten 204 bis 256 , Verlag Sauerländer Aarau und Frankfurt am Main, und hierin zitierte Referenzen, sowie Erich Ziegler "Die natürlichen und künstlichen Aromen" (1982), insbesondere Seiten 221 bis 242 , Dr. Alfred Hüthig Verlag Heidelberg, und hierin zitierte Referenzen.

Falls gewünscht, kann das erfindungsgemässe Verfahren am Ende des ersten Schrittes für eine gewünschte Zeitspanne unterbrochen werden. Der Primärextrakt kann an einem beliebigen Ort hergestellt, eingeengt, gegebenenfalls transportiert, und am Ort seiner Weiterverarbeitung zum gewünschten Zeitpunkt auf die jeweils gewünschte Konzentration rückverdünnt und dann nach dem erfindungsgemässen Verfahren weiterverarbeitet werden.

Die Adsorberharze, welche im Handel erhältlich sind, können mehrmals wiederverwendet werden.

Bevorzugte Adsorberharze haben eine Oberfläche von 100 bis 1000 m² pro Gramm und eine Korngrösse von 0,2 mm bis 1,5 mm.

Das erfindungsgemässe Verfahren hat zudem den grossen Vorteil, dass damit unerwünschte Begleitstoffe, wie etwa Stärke, Zucker, und insbesondere Rückstände von Pestiziden, wie Insektizide, Herbizide, Fungizide, etc., abgetrennt werden.

Die erfindungsgemäss hergestellten Wirkstoffkonzentrate können in Produkten der Pharma-, Kosmetik-und Nahrungsmittelindustrie verwendet werden.

Die nachfolgenden Beispiele illustrieren die vorliegende Erfindung.

### Beispiel 1

### Beschreibung zur Herstellung eines hochgehaltigen Ginkgo bilobae Extraktes

### a) Extraktion

35,0 kg Ginkgo bilobae Blätter wurden in den Extraktor eingefüllt und im Perkolationsverfahren mit der 10-fachen Menge einer Mischung von 12 % m/m Ethanol-Wasser und 1 % Ammoniumcarbonat bei einer Temperatur von 70°C bis zur Erschöpfung extrahiert. Der gemessene pH-Wert des Extraktionsmittels betrug ca. 8,4.

Man erhielt als Zwischenprodukt 350 kg eines Primärextraktes.

### b) Adsorbtion und Desorbtion

Der Primärextrakt wurde über eine Säule, die mit 100 kg Adsorberharz Amberlite XAD 16 von Röhm und Haas beladen war, gepumpt. Die Temperatur des Primärextraktes lag zwischen 15°C und 25°C. Die Durchströmmenge betrug zwischen 400 - 800 Liter pro Stunde.

Die Pflanzeninhaltsstoffe wurden nun vom Harz adsorbiert und die abströmende Restlösung wurde verworfen.

Nach einer Zwischenspülung mit ca. 125 Liter destilliertem Wasser bei Raumtemperatur wurde mit 25 Liter Ethanol desorbiert.

Nachfolgend wurde mit 100 Liter destilliertem Wasser bei Raumtemperatur nachgewaschen.

Die Ethanolphase und das Spülwasser enthielten nun die desorbierten Pflanzeninhaltsstoffe.

Die Lösung wurde nun unter Vakuum bis zu einem Trockensubstanz (TS) Gehalt von ca. 60 % eingedampft.

Das Adsorberharz kann mehrfach wiederbenützt werden.

### c) Trocknung

Die obige erhaltene Lösung mit einem TS Gehalt von ca. 60 % wurde auf übliche Weise auf einem Sprühtrockungsturm mit einer Zulufttemperatur von 180°C bis 200°C zu einem Pulver getrocknet.

Man erhielt 1000 g eines braunen Pulvers.

Der Gehalt an Flavonglycosiden wurde gemäss "Hagers Handbuch der Pharmazeutischen Praxis" von R. Hänsel et. al., Drogen E-O, Band 5, Springer Verlag, Seite 276 mittels HPLC ermittelt.

### Beispiel 2

### Beschreibung zur Herstellung eines hochgehaltigen Crataegi Extraktes

### a) Extraktion

25,0 kg Crataegi folia cum flores wurden in den Extraktor eingefüllt und im Perkolationsverfahren mit der 10-fachen Menge einer Mischung von 60% m/m Ethanol-Wasser bei einer Temperatur von 60°C bis zur Erschöpfung extrahiert.

Man erhielt als Zwischenprodukt 250 kg eines Primärextraktes.

Das Ethanol wurde unter Vakuum nahezu vollständig abdestilliert. Der Rückstand wurde mit Wasser auf einen Trockensubstanzgehalt von ca. 10 % verdünnt.

### b) Adsorbtion

Das erhaltene Gemisch wurde über eine Säule, die mit 60 kg Adsorberharz Amberlite XAD 7 von Röhm und Haas beladen war, gepumpt. Die Temperatur der Lösung lag zwischen 15°C und 25°C. Die Durchströmmenge betrug zwischen 120 - 400 Liter pro Stunde.

Die Pflanzeninhaltsstoffe wurden nun vom Harz adsorbiert und die abströmende Restlösung wurde verworfen.

Nach einer Zwischenspülung mit ca. 65 Liter destilliertem Wasser bei Raumtemperatur wurde mit 18 Liter Ethanol desorbiert.

Nachfolgend wurde mit 65 Liter destilliertem Wasser nachgewaschen.

Die Ethanolphase und das Spülwasser enthielten nun die desorbierten Pflanzeninhaltsstoffe.

Die Lösung wurde nun unter Vakuum bis zu einem TS-Gehalt von ca. 60 % eingedampft.

Das Adsorberharz kann mehrfach wiederbenützt werden.

### c) Trocknung

Die obige erhaltene Lösung mit einem TS Gehalt von ca. 60 % wurde auf übliche Weise auf einem Sprühtrockungsturm mit einer Zulufttemperatur von 180°C bis 200°C zu einem Pulver getrocknet.

Man erhielt 1000 g eines braunen Pulvers.

Der Gehalt an Flavonoiden wurde nach Deutschem Arzneibuch Nr. 10 (DAB 10), als Hyperosid berechnet, mit 7,6 % ermittelt.

### Beispiel 3

### Beschreibung zur Herstellung eines hochgehaltigen Hippocastani e. semen Extraktes

### a) Extraktion

7,50 kg Semen hippocastani gemahlen wurden in den Extraktor eingefüllt und im Perkolationsverfahren mit der 10-fachen Menge einer Mischung von 70 % m/m Ethanol-Wasser bei einer Temperatur von 60°C bis zur Erschöpfung extrahiert.

Man erhielt als Zwischenprodukt 75 kg eines Primärextraktes.

Das Ethanol wurde unter Vakuum nahezu vollständig abdestilliert. Der Rückstand wurde mit Wasser auf einen Trockensubstanzgehalt von ca. 6 % verdünnt.

### b) Adsorbtion

Die erhaltene Mischung wurde über eine Säule, die mit 60 kg Adsorberharz Amberlite XAD 7 von Röhm und Haas beladen war, gepumpt. Die Temperatur der Lösung lag zwischen 15°C und 25°C. Die Durchströmmenge betrug zwischen 120-360 Liter pro Stunde.

Die Pflanzeninhaltsstoffe wurden nun vom Harz adsorbiert und die abströmende Restlösung wurde verworfen.

Nach einer Zwischenspülung mit ca. 65 Liter destilliertem Wasser bei Raumtemperatur wurde mit 18 Liter Ethanol desorbiert.

Nachfolgend wurde mit 65 Liter destilliertem Wasser nachgewaschen.

Die Ethanolphase und das Spülwasser enthielten nun die desorbierten Pflanzeninhaltsstoffe.

Die Lösung wurde nun unter Vakuum bis zu einer TS Konzentration von ca. 60 % eingedampft.

Das Adsorberharz kann mehrfach wiederbenützt werden.

### c) Trocknung

Die obige erhaltene Lösung mit einem TS Gehalt von ca. 60 % wurde auf übliche Weise auf einem Sprühtrockungsturm mit einer Zulufttemperatur von 180°C bis 200°C zu einem Pulver getrocknet.

Man erhielt 1000 g eines braunen Pulvers.

Der Gehalt an Triterpensaponinen wurde gemäss DAB 10, berechnet als Aescin, von grösser als 70 % ermittelt.

### Beispiel 4

### Beschreibung zur Herstellung eines hochgehaltigen Salicis Extraktes

### a) Extraktion

55,0 kg Cortex salicis wurden in den Extraktor eingefüllt und im Perkolationsverfahren mit der 10-fachen Menge einer Mischung von 58 % m/m Ethanol-Wasser bei einer Temperatur von 60°C bis zur Erschöpfung extrahiert.

Man erhielt als Zwischenprodukt 550 kg eines Primärextraktes.

Das Ethanol wurde unter Vakuum nahezu vollständig abdestilliert. Der Rückstand wurde mit Wasser auf einen Trockensubstanzgehalt von ca. 10 % verdünnt.

### b) Adsorbtion

Das erhaltene Gemisch wurde über eine Säule, die mit 60 kg Adsorberharz Dowex-XUS von Dow Chemicals beladen war, gepumpt. Die Temperatur der Lösung lag zwischen 15°C und 25°C. Die Durchströmmenge betrug zwischen 80-240 Liter pro Stunde.

Die Pflanzeninhaltsstoffe wurden nun vom Harz adsorbiert und die abströmende Restlösung wurde verworfen.

Nach einer Zwischenspülung mit ca. 65 Liter destilliertem Wasser bei Raumtemperatur wurde mit 20 Liter Ethanol desorbiert.

Nachfolgend wurde mit 65 Liter destilliertem Wasser nachgewaschen.

Die Ethanolphase und das Spülwasser enthielten nun die desorbierten Pflanzeninhaltsstoffe.

Die Lösung wurde nun unter Vakuum bis zu einer TS Konzentration von ca. 60 % eingedampft.

Das Adsorberharz kann mehrfach wiederbenützt werden.

### c) Trocknung

Die obige erhaltene Lösung mit einem TS Gehalt von ca. 60 % wurde auf übliche Weise auf einem Sprühtrockungsturm mit einer Zulufttemperatur von 180°C bis 200°C zu einem Pulver getrocknet.

Man erhielt 1000 g eines braunen Pulvers.

Der Gehalt an Salicin [Bestimmt nach B. Meier und O. Sticher, Phärm. Acta Helv. 60, 269 (1985)] kann mit über 30 % ermittelt werden.

### Beispiel 5

### Beschreibung zur Herstellung eines hochgehaltigen Ginseng Extraktes

### a) Extraktion

40,0 kg Ginseng radix wurden in den Extraktor eingefüllt und im Perkolationsverfahren mit der 10-fachen Menge einer Mischung von 30 % m/m Ethanol-Wasser bei einer Temperatur von 70°C bis zur Erschöpfung extrahiert.

Man erhielt als Zwischenprodukt 400 kg eines Primärextraktes.

Das Ethanol wurde unter Vakuum bis auf einen Restgehalt von 15% m/m abdestilliert. Der Rückstand wurde mit Wasser auf einen Trockensubstanzgehalt von ca. 10 % verdünnt.

### b) Adsorbtion

Das erhaltene Gemisch wurde über eine Säule, die mit 60 kg Adsorberharz Amberlite XAD 7 von Röhm und Haas beladen war, gepumpt. Die Temperatur der Lösung lag zwischen 15°C und 25°C. Die Durchströmmenge betrug zwischen 100-240 Liter pro Stunde.

Die Pflanzeninhaltsstoffe wurden nun vom Harz adsorbiert und die abströmende Restlösung wurde verworfen.

Nach einer Zwischenspülung mit ca. 55 Liter destilliertem Wasser bei Raumtemperatur wurde mit 20 Liter Ethanol desorbiert.

Nachfolgend wurde mit 55 Liter destilliertem Wasser nachgewaschen.

Die Ethanolphase und das Spülwasser enthielten nun die desorbierten Pflanzeninhaltsstoffe.

Die Lösung wurde nun unter Vakuum bis zu einer TS Konzentration von ca. 60 % eingedampft.

Das Adsorberharz kann mehrfach wiederbenützt werden.

### c) Trocknung

Die obige erhaltene Lösung mit einem TS Gehalt von ca. 60 % wurde auf übliche Weise auf einem Sprühtrockungsturm mit einer Zulufttemperatur von 180°C bis 200°C zu einem Pulver getrocknet.

Man erhielt 1000 g eines gelbbraunen Pulvers.

Der Gehalt an Ginsenosiden, bestimmt nach DAB 10, wurde mit über 70 % ermittelt.

Es wurden folgende Gehalte an Fungiziden im Ausgangsmaterial und im erhaltenen Produkt ermittelt.

| | Quintozen (Mikrogramm/kg) | Pentachloranilin (Mikrogramm/kg) |
|---|---|---|
| Radix Ginseng (Ausgangsmaterial) | 4900 | 1600 |
| Extrakt aus Radix Ginseng (erhaltenes Produkt) | 26 | 440 |

Obige Zahlen zeigen, dass mit dem erfindungsgemässen Verfahren die unerwünschten Rückstände von Pestiziden stark reduziert werden können.

## Patentansprüche

1. Verfahren zur Herstellung von pestizidarmen Wirkstoffkonzentraten aus Pflanzen, dadurch gekennzeichnet, dass man
- in einem ersten Schritt Pflanzen oder Teile davon, welche im frischen oder getrockneten Zustand eingesetzt werden können, mit wenigstens einem polaren Lösungsmittel bei einem gemessenen pH-Wert im Bereich von 3,2 bis 9,8 derart extrahiert, dass im erhaltenen Primärextrakt hauptsächlich die gewünschten Pflanzeninhaltsstoffe, begleitet von nicht gewünschten Pflanzeninhaltsstoffen, Begleitstoffen und Pestiziden, enthalten sind, wobei im Falle von Ginkgo folia das genannte polare Lösungsmittel wässrig ist und von 0 bis 20 Gew.-% an Alkohol(en) und/oder Keton(en) enthält,
- in einem zweiten Schritt den genannten Primärextrakt derart mit Adsorberharz(en) in Kontakt bringt, dass einerseits die gewünschten Pflanzeninhaltsstoffe daran adsorbiert werden, und andererseits die Lösung mit den nicht gewünschten Pflanzeninhaltsstoffen, den Begleitstoffen und Pestiziden vom beladenen Harz separiert wird,
- in einem dritten Schritt die gewünschten Pflanzeninhaltsstoffe vom genannten Harz mit wenigstens einem Lösungsmittel desorbiert werden, und
- in einem vierten Schritt das (die) Lösungsmittel entweder teilweise oder vollständig entfernt und so ein Wirkstoffkonzentrat erhält.

2. Verfahren nach Anspruch 1, dadurch gekennzeichnet, dass das polare Lösungsmittel zur Herstellung des Primärextraktes ausgewählt ist aus der Gruppe, bestehend aus
- Wasser,
- einem Alkohol, insbesondere einem C₁ bis C₄-Alkohol, vorzugsweise Aethanol oder Methanol, und
- einem Keton, insbesondere einem C₃ bis C₅-Keton, vorzugsweise Aceton,
einschliesslich beliebige Gemische davon.

3. Verfahren nach einem der Ansprüche 1 bis 2, dadurch gekennzeichnet, dass der im ersten Schritt erwähnte pH-Wert eingestellt wird mittels der Hinzugabe wenigstens einer Säure oder Base, insbesondere ausgewählt aus der Gruppe, bestehend aus organischen Carbonsäuren, beispielsweise Ameisensäure, Essigsäure, Weinsäure, Zitronensäure, Ascorbinsäure, und anorganischen Basen, beispielsweise verdünnte wässrige Lösungen von Natriumhydroxid, Kaliumhydroxid, Carbonaten, wie etwa Natriumcarbonat, Kaliumcarbonat und Ammoniumcarbonat, und Hydrogencarbonaten, wie etwa Natriumhydrogencarbonat, Kaliumhydrogencarbonat und Ammoniumhydrogencarbonat.

4. Verfahren nach einem der Ansprüche 1 bis 3, dadurch gekennzeichnet, dass der mit dem Adsorberharz in Kontakt zu bringende Primärextrakt von 0 bis 30 Gew.-%, insbesondere von 10 bis 20 Gew.-%, bezogen auf den Primärextrakt, an Alkohol(en) und/oder Keton(en) enthält.

5. Verfahren nach einem der Ansprüche 1 bis 4, dadurch gekennzeichnet, dass der Primärextrakt bei einer Temperatur von 5°C bis 50°C, insbesondere von 15°C bis 30°C, mit dem Adsorberharz in Kontakt gebracht wird.

6. Verfahren nach einem der Ansprüche 1 bis 5, dadurch gekennzeichnet, dass die Adsorberharze unpolare oder polare Harze sind, wie Polyamide, Polyvinylpyrrolidone, insbesondere Polystyrol- und Acrylester-Harze, vorzugsweise mit einer Oberfläche von 100 bis 1000 m² pro Gramm an Adsorberharz, und mit einer Korngrösse von 0,2 mm bis 1,5 mm.

7. Verfahren nach einem der Ansprüche 1 bis 6, dadurch gekennzeichnet, dass das beladene Harz zwischen dem zweiten und dem dritten Schritt mit wenigstens einem Lösungsmittel gewaschen wird, insbesondere mit Wasser oder mit wässrigen Mischungen, welche bis zu 60 Gew.-%, insbesondere von 10 bis 20 Gew.-%, bezogen auf die gesamte Menge an Lösungsmittel, an Alkohol(en), insbesondere ein C₁ bis C₄-Alkohol, vorzugsweise Aethanol oder Methanol, und/oder Keton(en), insbesondere ein C₃ bis C₅-Keton, vorzugsweise Aceton, enthalten.

8. Verfahren nach einem der Ansprüche 1 bis 7, dadurch gekennzeichnet, dass das Waschen zwischen dem zweiten und dem dritten Schritt bei einer Temperatur von 5°C bis 50°C, insbesondere von 15°C bis 30°C, erfolgt.

9. Verfahren nach einem der Ansprüche 1 bis 8, dadurch gekennzeichnet, dass im dritten Schritt das Lösungsmittel zur Desorption der gewünschten Stoffe ausgewählt ist aus der Gruppe, bestehend aus
- Wasser und einer Säure oder Base, oder Säure und Base im Sinne eines Puffers, pH-Wert 0-14, insbesondere 1-3, oder 9-12,
- einem Alkohol, insbesondere einem C₁ bis C₆-Alkohol, vorzugsweise Aethanol oder Methanol, und
- einem Keton, insbesondere einem C₃ bis C₅-Keton, vorzugsweise Aceton,
einschliesslich beliebige Gemische davon mit einem Wasseranteil von 0 bis 100 Gew.-%, insbesondere von 10 bis 20 Gew.-%, bezogen auf die gesamte Menge an Lösungsmittel.

10. Verfahren nach einem der Ansprüche 1 bis 9, dadurch gekennzeichnet, dass das Desorptionsmittel Wasserdampf ist.

11. Verfahren nach einem der Ansprüche 1 bis 10, dadurch gekennzeichnet, dass die Desorption der gewünschten Stoffe bei einer Temperatur von 5°C bis 160°C, insbesondere von 15°C bis 100°C, vorzugsweise von 15°C bis 30°C, erfolgt.

12. Verfahren nach einem der Ansprüche 1 bis 11, dadurch gekennzeichnet, dass im vierten Schritt das (die) Lösungsmittel mittels Destillation teilweise oder vollständig entfernt wird (werden), vorzugsweise unter reduziertem Druck.

13. Verfahren nach einem der Ansprüche 1 bis 12, dadurch gekennzeichnet, dass die Pflanzen ausgewählt sind aus Alkaloide und/oder Flavonoide und/oder Saponine und/oder Bitterstoffe und/oder Terpene enthaltenden Pflanzen, insbesondere ausgewählt aus der Gruppe, bestehend aus
Boldo folia
Spartii radix,herba
Chinae cortex
Chelidonii herba
Chrysanthemum parthenium
Rauwolfiae radix
Betulae folia
Crataegi folia
Solidaginis herba
Passiflorae herba
Tiliae flores
Violae tricol. herba
Ginkgo folia
Ginseng radix
Senegae radix
Hippocastani semen
Hederae helic. herba
Liquiritiae radix
Gentianae radix
Salicis cortex.

14. Verfahren nach einem der Ansprüche 1 bis 13, dadurch gekennzeichnet, dass das am Ende des vierten Schrittes erhaltene Wirkstoffkonzentrat in ein Pulver übergeführt wird, beispielsweise mittels Sprühtrocknung.

15. Verfahren nach einem der Ansprüche 1 bis 14, dadurch gekennzeichnet, dass man zwischen dem ersten Schritt und dem zweiten Schritt gegebenenfalls vorhandene organische Lösungsmittel wenigstens teilweise entfernt, insbesondere mittels Destillation unter reduziertem Druck.

16. Verfahren nach einem der Ansprüche 1 bis 15 zur Herstellung eines pestizidarmen und allergenarmen Wirkstoffkonzentrates aus Ginkgo folia, dadurch gekennzeichnet, dass der Primärextrakt aus frischen oder getrockneten Ginkgo Blättern bei einem gemessenen pH-Wert im Bereich von 7,1 bis 9,8 hergestellt wird.

## Claims

1. A process for the preparation of pesticide-poor concentrates of active components of plants, characterized in that
- in a first step plants or parts thereof, which may be charged in fresh or dried state, are extracted with at least one polar solvent at a measured pH-value in the range from 3.2 to 9.8 in such a way, that in the obtained primary extract are contained mainly the desired plant components, accompanied by undesired plant components, accompanying substances and pesticides, whereby in the case of ginkgo folia said polar solvent is aqueous and contains from 0 to 20 % by weight of alcohol(s) and/or ketone(s),
- in a second step said primary extract is contacted with adsorber resin(s) in such a way, that on one hand the desired plant components are adsorbed thereon, and on the other hand the solution with the undesired plant components, the accompanying substances and pesticides is separated from the charged resin,
- in a third step the desired plant components are desorbed from said resin with at least one solvent, and
- in a fourth step the solvent(s) is (are) removed either partially or completely, and by this way a concentrate of active components is obtained.

2. The process according to claim 1, characterized in that the polar solvent for the preparation of the primary extract is selected from the group, consisting of
- water
- an alcohol, especially a C₁ to C₄-alcohol, preferably ethanol or methanol, and
- a ketone, especially a C₃ to C₅-ketone, preferably acetone,
including any mixtures thereof.

3. The process according to one of claims 1 to 2, characterized in that the pH-value mentioned in the first step is adjusted by means of the addition of at least one acid or base, especially selected from the group, consisting of organic carboxylic acids, for example formic acid, acetic acid, tartaric acid, citric acid, ascorbic acid, and inorganic bases, for example diluted aqueous solutions of sodium hydroxide, potassium hydroxide, carbonates, such as sodium carbonate, potassium carbonate and ammonium carbonate, and hydrogen carbonates, such as sodium hydrogen carbonate, potassium hydrogen carbonate and ammonium hydrogen carbonate.

4. The process according to one of claims 1 to 3, characterized in that the primary extract, to be contacted with the adsorber resin, contains from 0 to 30 % by weight, especially from 10 to 20 % by weight, referred to the primary extract, of alcohol(s) and/or ketone(s).

5. The process according to one of claims 1 to 4, characterized in that the primary extract is contacted with the adsorber resin at a temperature from 5°C to 50°C, especially from 15°C to 30°C.

6. The process according to one of claims 1 to 5, characterized in that the adsorber resins are non-polar or polar resins, such as polyamides, polyvinylpyrrolidones, especially polystyrene- and acrylester-resins, preferably with a surface from 100 to 1000 m² per gram of adsorber resin, and with a grain size from 0.2 mm to 1.5 mm.

7. The process according to one of claims 1 to 6, characterized in that between the second and the third step the charged resin is washed with at least one solvent, especially with water or with aqueous mixtures, which contain up to 60 % by weight, especially from 10 to 20 % by weight, referred to the total amount of solvent, of alcohol(s), especially a C₁ to C₄-alcohol, preferably ethanol or methanol, and/or ketone(s), especially a C₃ to C₅-ketone, preferably acetone.

8. The process according to one of claims 1 to 7, characterized in that the washing between the second and the third step is carried out at a temperature from 5°C to 50°C, especially from 15°C to 30°C.

9. The process according to one of claims 1 to 8, characterized in that in the third step the solvent for the desorption of the desired components is selected from the group, consisting of
- water and an acid or base, or acid and base in the sense of a buffer, pH-value 0-14, especially 1-3, or 9-12,
- an alcohol, esecially a C₁ to C₆-alcohol, preferably ethanol or methanol, and
- a ketone, especially a C₃ to C₅-ketone, preferably acetone,
including any mixtures thereof with a water portion from 0 to 100 % by weight, especially from 10 to 20 % by weight, referred to the total amount of solvent.

10. The process according to one of claims 1 to 9, characterized in that the desorption agent is water vapor.

11. The process according to one of claims 1 to 10, characterized in that the desorption of the desired components is carried out at a temperature from 5°C to 160°C, especially from 15°C to 100°C, preferably from 15°C to 30°C.

12. The process according to one of claims 1 to 11, characterized in that in the fourth step the solvent(s) is (are) removed partially or completely by means of distillation, preferably under reduced pressure.

13. The process according to one of claims 1 to 12, characterized in that the plants are selected from alkaloids and/or flavonoids and/or saponins and/or bitterings and/or terpenes containing plants, especially selected from the group, consisting of
Boldo folia
Spartii radix,herba
Chinae cortex
Chelidonii herba
Chrysanthemum parthenium
Rauwolfiae radix
Betulae folia
Crataegi folia
Solidaginis herba
Passiflorae herba
Tiliae flores
Violae tricol. herba
Ginkgo folia
Ginseng radix
Senegae radix
Hippocastani semen
Hederae helic. herba
Liquiritiae radix
Gentianae radix
Salicis cortex.

14. The process according to one of claims 1 to 13, characterized in that the at the end of the fourth step obtained concentrate of active components is transformed into a powder, for example by means of spray drying.

15. The process according to one of claims 1 to 14, characterized in that between the first and the second step occasionally present organic solvents are removed at least partially, especially by means of distillation under reduced pressure.

16. The process according to one of claims 1 to 15 for the preparation of a pesticide-poor and allergen-poor concentrate of active components of ginkgo folia, characterized in that the primary extract is prepared of fresh or dried ginkgo leafs at a measured pH-value in the range from 7.1 to 9.8.

## Revendications

1. Procédé de préparation de concentrés d'agents actifs pauvres en pesticides en provenance de plantes, caractérisé en ce que l'on :
- extrait dans une première étape, les plantes ou des parties de celles-ci, lesquelles peuvent être mises en oeuvre à l'état frais ou séché, avec au moins un solvant polaire à un pH mesuré dans la plage allant de 3,2 à 9,8, en ce que dans l'extrait primaire obtenu, sont présents essentiellement les constituants végétaux souhaités, accompagnés de constituants végétaux non souhaités, d'impuretés et de pesticides, où dans le cas de Ginkgo folia, le solvant polaire cité est aqueux et contient 0 à 20% en poids d'un ou de plusieurs alcools et/ou d'une ou de plusieurs cétones ;
- met en contact, dans une deuxième étape, l'extrait primaire indiqué avec une ou plusieurs résines adsorbantes, de sorte que d'une part, les constituants végétaux souhaités y soient adsorbés et d'autre part, que le solvant avec les constituants végétaux non souhaités, les impuretés et les pesticides soit séparé de la résine chargée ;
- désorbe dans une troisième étape, les constituants végétaux souhaités de la résine indiquée avec au moins un solvant, et
- élimine dans une quatrième étape, partiellement ou complètement, le ou les solvants et on obtient ainsi, un concentré d'agents actifs.

2. Procédé suivant la revendication 1, caractérisé en ce que le solvant polaire pour la préparation de l'extrait primaire est choisi parmi le groupe consistant en :
- l'eau ;
- un alcool, en particulier un alcool en C₁-C₄, de préférence l'éthanol ou le méthanol, et
- une cétone, en particulier une cétone en C₃-C₅, de préférence l'acétone,
y compris les mélanges quelconques de ceux-ci.

3. Procédé suivant l'une quelconque des revendications 1 à 2, caractérisé en ce que le pH mentionné dans la première étape est ajusté par addition d'au moins un acide ou base, en particulier choisis parmi le groupe consistant en les acides carboxyliques organiques, par exemple l'acide formique, l'acide acétique, l'acide tartrique, l'acide citrique, l'acide ascorbique et les bases inorganiques, par exemple des solutions aqueuses diluées d'hydroxyde de sodium, d'hydroxyde de potassium, de carbonates, comme le carbonate de sodium, le carbonate de potassium et le carbonate d'ammonium et d'hydrogénocarbonates, comme l'hydrogénocarbonate de sodium, l'hydrogénocarbonate de potassium et l'hydrogénocarbonate d'ammonium.

4. Procédé suivant l'une quelconque des revendications 1 à 3, caractérisé en ce que l'extrait primaire à mettre en contact avec la résine adsorbante contient de 0 à 30% en poids, en particulier de 10 à 20% en poids, sur base de l'extrait primaire, d'un ou plusieurs alcools et/ou d'une ou plusieurs cétones.

5. Procédé suivant l'une quelconque des revendications 1 à 4, caractérisé en ce que l'extrait primaire est mis en contact avec la résine adsorbante à une température allant de 5°C à 50°C, en particulier de 15°C à 30°C.

6. Procédé suivant l'une quelconque des revendications 1 à 5, caractérisé en ce que les résines adsorbantes sont des résines polaires ou non polaires, comme un polyamide, une polyvinylpyrrolidone, en particulier une résine polystyrène ou acrylate, de préférence avec une surface allant de 100 à 1000 m² par gramme de résine adsorbante, et avec une taille des particules allant de 0,2 mm à 1,5 mm.

7. Procédé suivant l'une quelconque des revendications 1 à 6, caractérisé en ce que la résine chargée entre les deuxième et troisième étapes, est lavée avec au moins un solvant, en particulier avec de l'eau ou des mélanges aqueux qui contiennent jusqu'à 60% en poids, en particulier de 10 à 20% en poids, sur base de la quantité totale de solvant, d'un ou plusieurs alcools, en particulier un alcool en C₁-C₄, de préférence l'éthanol ou le méthanol, et/ou une ou plusieurs cétones, en particulier une cétone en C₃-C₅, de préférence l'acétone.

8. Procédé suivant l'une quelconque des revendications 1 à 7, caractérisé en ce que le lavage entre les deuxième et troisième étapes, est réalisé à une température allant de 5°C à 50°C, en particulier de 15°C à 30°C.

9. Procédé suivant l'une quelconque des revendications 1 à 8, caractérisé en ce que dans la troisième étape, le solvant pour la désorption des substances souhaitées est choisi parmi le groupe consistant en :
- l'eau et un acide ou une base, ou un acide et une base dans le sens d'un tampon, pH 0-14, en particulier 1-3 ou 9-12 ;
- un alcool, en particulier un alcool en C₁-C₆, de préférence l'éthanol ou le méthanol, et
- une cétone, en particulier une cétone en C₃-C₅, de préférence l'acétone,
y compris les mélanges quelconques de ceux-ci, avec une teneur en eau allant de 0 à 100% en poids, en particulier de 10 à 20% en poids, sur base de la quantité totale de solvant.

10. Procédé suivant l'une quelconque des revendications 1 à 9, caractérisé en ce que l'agent de désorption est la vapeur d'eau.

11. Procédé suivant l'une quelconque des revendications 1 à 10, caractérisé en ce que la désorption des substances souhaitées est réalisée à une température allant de 5°C à 160°C, en particulier de 15°C à 100°C, de préférence de 15°C à 30°C.

12. Procédé suivant l'une quelconque des revendications 1 à 11, caractérisé en ce que dans la quatrième étape, le ou les solvants sont partiellement ou complètement éliminés par distillation, de préférence sous pression réduite.

13. Procédé suivant l'une quelconque des revendications 1 à 12, caractérisé en ce que les plantes sont choisies parmi les plantes contenant des alcaloïdes et/ou des flavonoïdes et/ou des saponines et/ou des substances amères et/ou des terpènes, en particulier choisies parmi le groupe consistant en :
Boldo folia,
Spartii radix, herba,
Chinae cortex,
Chelidonii herba,
Chrysanthenum parthenium,
Rauwolfiae radix,
Betulae folia,
Crataegi folia,
Solidaginis herba,
Passiflorae herba,
Tiliae flores,
Violae tricol. herba,
Ginkgo folia,
Ginseng radix,
Senegae radix,
Hippocastani semen,
Hederae helic. herba,
Liquiritiae radix,
Gentianae radix,
Salicis cortex.

14. Procédé suivant l'une quelconque des revendications 1 à 13, caractérisé en ce que le concentré d'agents actifs obtenu à la fin de la quatrième étape est traité en poudre, par exemple par séchage par pulvérisation.

15. Procédé suivant l'une quelconque des revendications 1 à 14, caractérisé en ce que l'on élimine, entre les première et deuxième étapes, au moins partiellement le solvant organique facultativement présent, en particulier par distillation sous pression réduite.

16. Procédé suivant l'une quelconque des revendications 1 à 15, de préparation de concentrés d'agents actifs pauvres en pesticides et pauvres en allergènes de Ginkgo folia, caractérisé en ce que l'extrait primaire est préparé à partir de feuilles de Ginkgo fraîches ou séchées à un pH mesuré dans la plage allant de 7,1 à 9,8.
